# EUROPEAN PATENT APPLICATION

(11) **EP 1 788 083 A1**
(43) Date of publication of application: **23.05.2007**
(21) Application number: 05768326.0
(22) Date of filing: 02.08.2005
(51) Int. Cl.: C12N 15/09, C07K 16/18, C12Q 1/02, C12Q 1/68, A61K 45/00, A61P 35/00

(54) **METHOD OF ASSUMING DRUG SENSITIVITY TO CDK4 INHIBITOR**

(30) Priority: 02.08.2004 JP 2004226033
(71) Applicant: BANYU PHARMACEUTICAL CO., LTD., Tokyo 102-8667 (JP)
(72) Inventor: KOTANI, Hidehito, c/o Tsukuba Research Institute, Tsukuba-shi, Ibaraki 3002611 (JP); ITADANI, Hiraku, c/o Tsukuba Research Institute, Tsukuba-shi, Ibaraki 3002611 (JP); YAMANAKA, Kazunori, c/o Tsukuba Research Institute, Tsukuba-shi, Ibaraki 3002611 (JP); EGUCHI, Tomohiro, c/o Tsukuba Research Institute, 3, Okubo, Tsukuba-shi, Ibaraki 3002611 (JP); SHIMOMURA, Toshiyasu, Tsukuba Research Institute, 3, Okubo, Tsukuba-shi, Ibaraki 3002611 (JP)
(74) Representative: Horgan, James Michael Frederic
(86) International application number: PCT/JP2005/014123
(87) International publication number: WO 2006/013862

(57) **Abstract**

It is intended to provide a gene for assuming drug sensitivity, which is to be used for assuming the drug sensitivity to a CDK4 inhibitor based on the expression amount in a test tissue or a test cell, selected from the group consisting of p16 gene, p18 gene and p27 gene. It is also intended to provide a method of assuming the drug sensitivity to a CDK4 inhibitor by using the expression amount of the above gene in a test tissue or a test cell as an indication.

## Description

### Technical Field

The present invention relates to a method for measuring and assuming the sensitivity to a CDK4 inhibitor in a test tissue or a test cell on a molecular level. It also relates to a molecule used for said measurement and assumption.

### Background Art

Due to the progress of human genome project in recent years, pharmacogenomics where differences in drug sensitivity among individuals are understood on a gene level and to apply it to development of drugs has been in a quick progress. If differences among individuals in drug sensitivity are able to be judged on a gene level, it is then possible to conduct the so-called made-to-order therapy where a drug having a strong side effect is administered only to a patient with whom the pharmaceutical effect can be really expected or to conduct a suitable drug administration from the initial stage of the treatment without carrying out the trial-and-error administration. As a result, useless physical burden is no longer applied to patients and, in addition, that contributes in reduction in medical expenses which tend to significantly increase in recent years. Particularly, anticancer agents often have very strong side effects and there has been a brisk demand for a made-to-order therapy.

On the other hand, abnormality in cell cycle is listed as one of the causes for carcinogenesis. Cell cycle is a fundamental system of cell division and serves in the final stage of signal transduction in cell growth control. Cell cycles proceeds in such a manner that the state where cell growth stops is G0 phase and then in the order of G1 phase, S phase, G2 phase and M phase. In the S phase, duplication of DNA is conducted and, in the M phase, cell division is conducted. Various proteins are participated in the control of the cell cycle and, among them, a cyclin-CDK (a cyclin-dependent kinase) complex plays a leading role. Thus, cyclin D or cyclin E and cyclin A is/are activated by forming a complex with CDK4 (or CDK6) or with CDK2, respectively. The activated complex phosphorylates Rb (retinoblastoma) protein to progress the signal transduction and transcription of E2F, etc. is activated whereby the cell is induced from G1 phase to S phase.

Hereinabove, CDK4 (accession number for human CDK4 gene: U37022; SEQ ID No. 1) is a protein of 34 kD comprising 303 amino acids and has been known as a gene which neutralizes the growth suppression activity of Rb protein by phosphorylation thereof and has been reported to be highly expressed in some types of cancer cells (Adv. Cancer Res., 1996, volume 68, page 67).

With regard to a CDK inhibitor, plural types thereof have been known and are roughly classified into a Cip/Kip family having homology to p21 and an INK4 family having homology to p16. Any of them inhibits the phosphorylation activity of the cyclin-CDK complex and suppresses phosphorylation of Rb protein to inhibit the process of cell cycle but there is a difference in action mechanisms between them in such a respect that, for example, the Cip/Kip family bonds to plural cyclin-CDK complexes to inhibit them while the INK4 family bonds only to CDK4 or CDK6 to inhibit it.

As an example of the Cip/Kip family, p27 (accession number of human p27 gene: NM_004064; SEQ ID No. 2) is listed. The p27 has been identified as a protein of about 27 kDa bonding to a cyclin E-CDK2 complex in cells where growth stops inhibiting the activity thereof (Genes Dev., 1994, volume 8, page 9).

Further, as examples of the INK4 family, p16 (accession number of human p16 gene: NM_000077; SEQ ID No. 3) and p18 (accession number of human p18 gene: NM_001262; SEQ ID No. 4) are listed. With regard to the p16 bonding to CDK4, cDNA has been isolated as a CDK inhibitor which inhibits its activity *(*Nature, 1993, volume 366, page 704). The p16 is a 16-kDa protein comprising 156 amino acids. On the other hand, the p18 has been cloned by investigating a protein interacting to CDK6 by an enzyme two-hybrid system (Genes Dev., 1994, volume 8, page 2939). Guan, et al. have found that the p18 interacts to CDK6 strongly and to CDK4 gently while it does not interact to other CDK at all.

Incidentally, as mentioned above, the made-for-order therapy, etc. will significantly progress if the differences in drug sensitivity among individuals are able to be judged on a gene level. Such an attempt has been carried out already and, in the field of cancer, there is a report where DNA microarray is used and sensitivity and resistance to anticancer agents are investigated where expression of many genes is an indicator (Cancer Res., 2001, volume 61, page 6474; Non-Patent Document 1). In addition, John M. Mariadason, et al. have investigated the drug sensitivity of cancer cells to 5-FU (5-fluorouracil) and CPT (camptothecin) using expression amount of various kinds of gene as an indicator (Cancer Res., 2003, volume 63, page 8791; Non-Patent Document 2).
Non-Patent Document: Cancer Research, 2001, volume 61, page 6474
Non-Patent Document: Cancer Research, 2003, volume 63, page 8791

### Disclosure of the Invention

### Problems that the Invention is to Solve

However, in Non-Patent Documents 1 and 2, there is no description to the effect that sensitivity and resistance to drugs are able to be assumed using expression amount of p16, p18 or p27 as an indicator and there is also no suggestion at all for the relation between the expression amount of such genes and drug sensitivity and pharmaceutical effect to the CDK4 inhibitors.

The present invention has been achieved in view of the above-mentioned problems in the prior art and its object is to provide the gene which is used as an indicator in the assumption of degree of drug sensitivity of a CDK4 inhibitor. Another object is to provide a method of assuming the drug sensitivity to a CDK4 inhibitor using the expression amount of such a gene.

### Means for Solving the Problems

The present inventors have repeatedly carried out intensive studies for achieving the above-mentioned problems and found that expression amount of p16, p18 or p27 which has been known as a molecule for controlling the cell cycle as a CDK4 inhibitor is able to act as an indicator for assuming the presence or the absence of the drug sensitivity to CDK4 inhibitor or for assuming the degree thereof whereupon the present invention has been achieved.

Thus, the present invention provides a gene for assuming drug sensitivity, which is to be used for assuming the drug sensitivity to a CDK4 inhibitor based on the expression amount in a test tissue or a test cell, selected from the group consisting ofp16 gene, p18 gene and p27 gene. Since the CDK4 inhibitor has an action of suppressing the phosphorylation of Rb protein to suppress the cell growth, it is useful as a treating drug for proliferative diseases and, on the other hand, it has a side action to suppress the growth of healthy cells as well. However, when drug sensitivity is assumed using the above-mentioned gene for assumption of drug sensitivity prior to administration of the CDK4 inhibitor to a patient, administration of the drug to a patient for whom the CDK4 inhibitor is ineffective can be avoided whereby the side action can be greatly reduced.

The above-mentioned gene for assumption of the drug sensitivity is preferred to be a p18 gene. Since the p18 gene is significantly correlated to drug sensitivity to a CDK4 inhibitor, more accurate assumption is possible.

The present invention also provides a gene for assuming drug sensitivity, which is to be used for assuming the drug resistance to a CDK4 inhibitor based on the expression amount in a test tissue or a test cell, selected from the group consisting of p16 gene, p18 gene and p27 gene. Those genes for assumption of drug sensitivity are also able to be advantageously used for assumption of not only drug per-sensitivity but also drug resistance to the CDK4 inhibitors.

The present invention further provides a reagent for detection of drug sensitivity to a CDK4 inhibitor containing an antibody which specifically recognizes an antisense nucleic acid of gene selected from the group consisting of p16 gene, p18 gene and p27 gene or protein expressed by said gene. When the above-mentioned reagent for detection of drug sensitivity is used, it is possible to assume the sensitivity to CDK4 inhibitor before administration of this drug to a patient. In the meanwhile, the protein in which p16 gene, p18 gene or p27 gene is expressed is represented by SEQ ID No. 8, No. 9 or No. 10, respectively.

In assuming the drug sensitivity to the above CDK4 inhibitor, it is preferred to use a probe for detection of drug sensitivity comprising an antisense nucleic acid of gene selected from the group consisting of p16 gene, p18 gene and p27 gene or an antibody for detection of drug sensitivity which specifically recognizes the protein in which the above gene is expressed.

The present invention further provides a method of assuming the drug sensitivity to a CDK4 inhibitor including a step (measuring step) where expression amount of at least one gene selected from the group consisting of p16 gene, p18 gene and p27 gene in a test tissue or in a test cell is measured and a step (comparing step) where the expression amount of said gene is compared with the expression amount of said gene in a healthy tissue or in a healthy cell. When such a method is used, it is possible to assume the sensitivity to a CDK4 inhibitor before this drug is administered to a patient.

When a step (step for judging the high sensitivity) where it is judged that the drug sensitivity of a test tissue or a test cell to a CDK4 inhibitor is high is further included in the above-mentioned assuming method in case expression of the above gene in a test tissue or in a test cell is lower than expression of the above gene in a healthy tissue or in a healthy cell as a result of comparison of the expression amounts, then it is able to be assumed that a drug sensitivity to a CDK4 inhibitor is high in case expression of the above-mentioned gene is low in a test tissue or in a test cell as compared with that in a healthy tissue or in a healthy cell.

The present invention furthermore provides a method of assuming the drug sensitivity to a CDK4 inhibitor including a step (detecting step) where detection of expression of at least one gene selected from the group consisting of p16 gene, p18 gene and p27 gene in a test tissue or a test cell. When this method is used, both measuring method and comparing method are unnecessary and, therefore, it is now possible to easily assume the sensitivity to a DK4 inhibitor.

When a step (step for judging the low sensitivity) where a drug sensitivity of the above-mentioned test tissue or test cell to a CDK4 inhibitor is judged to be low is included in the above-mentioned assuming method in case the above-mentioned gene is expressed as a result of the detection, then it is able to be assumed that the drug sensitivity to a CDK4 inhibitor is low in case expression of the above-mentioned gene is detected in the test tissue or test cell.

Moreover, in the above-mentioned assuming method for the drug sensitivity, the gene where its expression is detected is preferred to be p 18 gene. In the p 18 gene, there is a significant correlation to the drug sensitivity to a CDK4 inhibitor and, therefore, more accurate assumption is possible in the above-mentioned assuming method.

### Advantages of the Invention

According to the method of assuming a drug sensitivity to a CDK4 inhibitor according to the present invention, there is provided a gene to be used as an indicator in assumption of degree of the drug sensitivity of a CDK4 inhibitor or, in other words, the use of p16, p18 or p27 (gene for assuming the drug sensitivity). There is also provided a method of assuming the drug sensitivity to a CDK4 inhibitor using expression amount of those genes.

Incidentally, p16, p18 and p27 each is a factor which controls the cell cycle to a negative side and is a product of a cancer-suppressive gene having an action of inhibiting the kinase activity of the corresponding CDK and, therefore, although an attempt of using the gene *per se* as a target for anticancer drugs, it has not been unable to assume up to now that those genes are able to be utilized for assuming the sensitivity of a CDK4 inhibitor achieving the same action as the CDK inhibitor.

### Brief Description of the Drawings

Fig. 1 is a drawing where changes in level of phosphorylation of Rb protein by a treatment with a compound A are confirmed.
Fig. 2 is a drawing where inhibition of cell growth by a compound A and induction of apoptosis are compared for incubated cells derived from various kinds of tissues.
Fig. 3 is a drawing where, for incubated cells derived from various kinds of tissues, gene where expression amount in those cells changes is detected by a microarray and subjected to a clustering.
Fig. 4 is a drawing where, for incubated cells derived from various kinds of tissues, expression amounts of p18, p16, p27 and phosphorylated Rb protein (pRB) are confirmed.
Fig. 5 is a drawing where induction of expression of p18 is confirmed in HeLa cells in which expression of p18 is induced.
Fig. 6 is a drawing which shows the relation between expression of p18 and apoptosis induction.
Fig. 7 is a drawing where gene in which expression amount changes by an excessive expression of p16 and p27 is checked at the four time points of 24, 36, 48 and 72 hours and plotted.
Fig. 8 is a drawing where gene in which expression amount changes by an excessive expression of p16 and p27 is confirmed at the four time points of 24, 36, 48 and 72 hours.
Fig. 9 is a drawing where gene in which expression amount changes by an excessive expression of p16 and p27 is detected at the four time points of 24, 36, 48 and 72 hours and subjected to clustering.

### Best Mode for Carrying Out the Invention

As hereunder, advantageous embodiments of the present invention will be illustrated in detail.

Firstly, the terms used in the present invention will be illustrated.

With regard to "test tissue", although there is no particular limitation for its type so far as it is a tissue derived from a sample (mostly, a human) which is to be measured for its sensitivity to a CDK4 inhibitor, it is preferred to be blood in view of easy separation and extraction. In blood, hemocyte is able to be considered as a blood component and plasma containing fibrinogen is able to be considered as an extracellular substrate and, therefore, blood is able to be grasped as a supporting tissue. In addition, since a target disease for a CDK4 inhibitor is mostly a proliferative disease (such as cancer, tumor and chronic articular rheumatism), the tissue is preferred to be a tissue derived from an organ where such a disease is discovered.

With regard to "test cell", although there is no particular limitation for its type so far as it is a cell derived from a sample (mostly, a human) which is to be measured for its sensitivity to a CDK4 inhibitor, it is preferred to be blood cell (not only hemocyte cell such as red blood cell and white blood cell but also a mixture thereof) in view of easy separation and extraction. In addition, since a target disease for a CDK4 inhibitor is mostly a proliferative disease (such as cancer, tumor and chronic articular rheumatism), the cell is preferred to be a cell derived from an organ where such a disease is discovered.

With regard to "CDK4 inhibitor", there is no particular limitation for its type so far as it is a substance which inhibits the activity of CDK4 and it may be either a low-molecular or a high-molecular compound or may be protein or peptide. It may be also a low-molecular physiologically active substance. Specific examples of the CDK4 inhibitor as such are flavopiridol, BMS 387032 and R-roscovitine.

With regard to "drug sensitivity", it inherently differs depending upon sex, age, nutritional state and pathology and is also affected by drug concentration in the tissue and, independently of such a factor, degree of sensitivity may be judged on the ground whether the drug is to be administered or is not. Thus, expression amount of p16, p18 or p27 gene is measured and it is to be checked whether the drug is to be administered depending upon the expression amount.

When drug sensitivity changes, there are some cases where drug resistance or drug hypersensitivity is noted. Drug resistance is usually a phenomenon where reactivity to the drug lowers as a result of administration of a drug either continuously or frequently within short time. With regard to a cause where drug resistance is achieved, there have been known, for example, a decrease in receptor numbers, a decrease in bonding affinity of receptor and a lowering in activity of information transmittance system. On the other hand, drug hypersensitivity is a phenomenon where, when agonist concentration at the acting site decreases for example, reactivity to an agonist which is given later significantly increases. With regard to a cause where drug hypersensitivity is achieved, there have been known, for example, an increase in receptor and an increase in information transmittance system from the receptor.

"Gene for assuming drug sensitivity" is p16, p18 or p27 gene used for assuming the drug sensitivity to a CDK4 inhibitor based on the expression amount in a test tissue or a test cell.

As illustrated in the column of the background art, p16, p18 or p27 gene has been known as a factor having an action of inhibiting the phosphorylating activity of CDK in living body. Any of those genes inhibits the phosphorylation of Rb protein by cyclin-CDK by bonding to a cyclin-CDK complex. Thus, the gene functions as a regulating factor for an Rb route. From those facts, it is presumed that activity of CDK is high and the Rb route is activated in cells where expression of p16, p18 or p27 gene is low and it is presumed that there is a correlation between the gene and the CDK activity although there has been no knowledge at all whether there is any relation between sensitivity to a CDK4 inhibitor and expression amount of p16, p18 or p27 gene. The present inventors have classified on a cell level or on a tissue level according to the difference in the sensitivity to a CDK4 inhibitor whereby they have clarified the correlation between the CDK4 inhibitor and expression amount of p16, p18 or p27 gene and found the use of the above-mentioned gene which assumes the drug sensitivity.

With regard to animal species which are sources of CDK4 and each gene of p16, p18 and p27, human and non-human animals are examples and, although human, mouse, rat, monkey, dog and rabbit are exemplified, human is particularly preferred.

In the present invention, use of p16, p 18 or p27 gene is disclosed and that which is particularly preferred for the use where a drug sensitivity to a CDK4 inhibitor is p18 gene. That is because the relation between its expression amount and drug sensitivity is significant.

"Probe for detection of drug sensitivity" to a CDK4 inhibitor means an antisense nucleic acid to gene selected from the group consisting of p16 gene, p18 gene and p27 gene and its use is for checking the drug sensitivity to a CDK4 inhibitor.

The above-mentioned antisense nucleic acid may be either an antisense nucleic acid of full or partial sequence of the gene or any of DNA and RNA so far as it is able to specifically recognize each mRNA of p16 gene, p18 gene and p27 gene expressed in a test tissue or a test cell. When mRNA of the above-mentioned gene is detected by a northern blotting or by a hybridization method such as an *in situ* hybridization, an antisense nucleic acid in the length of not shorter than 100 bp is usually used. However, it is preferably not shorter than 500 bp and, more preferably, not shorter than 1,000 bp. If, however, the corresponding mRNA is able to be specifically recognized, an oligonucleotide of about several tens bp may be acceptable.

The antisense nucleic acid is able to be synthesized by a PCR method using the above-mentioned gene as a template if it is DNA while, in case it is RNA, synthesis is possible by means of an *in vitro* translation method using the above-mentioned gene as a template. In the case of an oligonucleotide, synthesis is possible using a DNA synthesizer.

With regard to an antibody which specifically recognizes p16, p18 and p27, it may be either a monoclonal antibody or a polyclonal antibody so far as it is able to recognize each of p16, p18 and p27 expressed in a test tissue or a test cell. Incidentally, the above-mentioned antibody is able to be synthesized, for example, by immunizing the above-mentioned protein or synthetic peptide thereof to rabbits or by means of a hybridoma method.

"Reagent for detection of drug sensitivity" is a reagent for checking the drug sensitivity to a CDK4 inhibitor where the above-mentioned antisense nucleic acid or the above-mentioned antibody is an essential component and, although it may be solely composed of the above-mentioned antisense nucleic acid or the above-mentioned antibody, it is preferred to be a mixture of the above-mentioned antisense nucleic acid or the above-mentioned antibody with other component. Such a reagent for detecting the drug sensitivity is able to be manufactured in such a manner that the above-mentioned antisense nucleic acid or antibody is dissolved together with a reducing agent and an enzyme inhibitor preventing the decomposition thereof and, in addition, a constituting component which is necessary depending upon the use is contained therein.

Now "a method of assuming the drug sensitivity to a CDK4 inhibitor" concerning the first embodiment of the present invention will be illustrated.

"Method of assuming drug sensitivity to a CDK4 inhibitor" concerning the first embodiment is characterized in containing a measuring step where expression amount of at least one gene selected from the group consisting of p16, p18 and p27 in a test tissue or a test cell is measured and a comparing step where the expression amount of the above-mentioned gene is compared with the expression amount of the above-mentioned gene in a healthy tissue or in a healthy cell.

In the measuring step, expression amount of at least one gene among p16, p18 and p27 is measured in a test tissue or a test cell separated from a test sample. Here, "at least one" means that the expression amount may be measured only for one gene among p16, p18 and p27 or the expression amount may be measured for two or three genes thereof. Even when two or three genes are measured, it is preferred to measure the expression amount including at least p18 so as to conduct more accurate assumption. With regard to a method of measurement, although there is no particular limitation, it is possible, for example, that the test tissue or the test cell is solubilized and then analyzed by means of a DNA mciroarrary or the like whereby expression of such gene is measured. When a translation product of the above-mentioned gene is detected, expression amount of the translation product is able to be checked by, for example, a western blotting.

In a comparing step, expression amount of p16, p18 or p27 gene in a test tissue or a test cell measured in the measuring step is compared with expression amount of p16, p18 or p27 gene in a healthy tissue or a healthy cell separated from a healthy person. Although there is no particular limitation for the method of comparison, its specific examples are that, in the case of a DNA microarray, fluorescence intensity on the array is quantified while, in the case of a western blotting, detected band is numerically expressed by a densitometer or the like and then comparison is carried out.

In the first embodiment, a step of judging the hypersensitivity may be included in addition to the above-mentioned measuring step and comparing step. The step for judging the hypersensitivity is that, when expression of p16, p18 or p27 gene in a test tissue or a test cell is lower than the expression of said gene in a healthy tissue or a healthy cell as a result of comparison of expression amounts, then it is judged that the test tissue or the test cell has a high drug sensitivity to a CDK4 inhibitor.

When, in a step for judging the hypersensitivity, expression of the gene in a test tissue or a test cell is lower than that in a healthy tissue or a healthy cell as a result of comparison of expression amounts of the gene in a comparing step, then it is able to be assumed that a sample wherefrom said test tissue or said test cell is separated is highly sensitive to a CDK4 inhibitor.

Now, "method of assuming the drug sensitivity to a CDK4 inhibitor" which is the second embodiment of the present invention will be illustrated.

"Method of assuming the drug sensitivity to a CDK4 inhibitor" which is the second embodiment of the present invention is characterized in containing a detecting step where expression of at least one gene selected from the group consisting of p16, p18 and p27 in a test tissue or a test cell is detected.

A method of assuming the drug sensitivity to a CDK4 inhibitor according to the first embodiment of the present invention is that the degree of sensitivity to a CDK4 inhibitor is assumed by comparing the expression amounts of p16, p18 and p27 genes in a test tissue and a test cell with those in a healthy tissue or a healthy cell. However, those genes are able to be judged for their degree of sensitivity to a CDK4 inhibitor not only by means of comparing with healthy tissue but also just by means of confirming whether or not the expression is available. Thus, in a method of assuming according to the second embodiment, there is a tendency that, in the tissue and the cell having a high sensitivity, expression of the above-mentioned gene (particularly, p18) is low or there is no expression at all. Accordingly, when no expression is detected at all or, although the expression was noted, the expression amount is extremely low as a result of checking the expression of the above-mentioned gene, it is able to be assumed that the sensitivity to a CDK4 inhibitor is high.

Although there is no particular limitation for a method of detecting the expression of p 16, p18 and p27 genes, a specific example thereof is that detection is possible by a DNA microarray or the like after solubilizing a test tissue or a test cell. In the case of detection of a translation product of the above-mentioned gene, it is able to be checked whether or not the translation product is expressed by, for example, a western blotting.

In the second embodiment, expression of at least one gene from p16, p18 or p27 gene in a test tissue or a test cell separated from a test sample is detected and, with regard to "at least one" used here, expression amount may be measured for only one of p16, p18 and p27 or that may be measured for two or three genes. Even when two or three genes are measured, it is preferred to measure the expression amount including at least p18 so as to conduct more accurate assumption.

In the second embodiment, a step for judging the low sensitivity may be included in addition to the above-mentioned detecting step. The step for judging the low sensitivity is a step where, when the above-mentioned gene is expressed as a result of the detection, the above-mentioned test tissue or the test cell has low drug sensitivity to a CDK4 inhibitor.

When expression of gene is detected in a detecting step as a result of conducting the step for judging the low sensitivity, it is able to be assumed that the test sample wherefrom said test tissue or test cell is separated has a low sensitivity to a CDK4 inhibitor.

In accordance with the gene for assuming the drug sensitivity and with the method for assuming the drug sensitivity of the present invention as illustrated hereinabove, it is now possible to assume the sensitivity to a CDK4 inhibitor and, particularly, the knowledge concerning a drug resistance based on the expression amount of p16, p18 or p27 gene. The above means that, only by measurement of expression of p16, p18 or p27 gene in a test tissue or test cell such as blood separated from a test sample, it is now possible to assume the sensitivity to a CDK4 inhibitor to be administered and the pharmaceutical effect and the so-called made-to-order is now possible.

### Examples

### Example 1: Experiment for CDK4 inhibitor resistance

### (Construction of plasmid)

p18 Gene was amplified by a PCR method using cDNA of HeLa cells as a template. The amplified p18 gene was inserted into pCR 2.1-TOPO vector by a TOPO TA cloning kit (manufactured by Invitrogene) to construct an expression vector.

Further, pTRE2hyg FLAG-p18 vector was constructed by insertion of FLAG gene and p 18 gene into pTRE2hyg vector.

### (Method of cell culture)

All clinical cancer cell strains were incubated at 37°C in an environment of saturated water vapor in the presence of 5% CO₂. HCT 116, SCC-25, PA-1 and U-118 MG were incubated using a D-MEM medium (manufactured by Gibco) to which 10% of fetal bovine serum was added. PC 13, MSTO-211H, BxPC-3 and NCI-H69 were incubated using an RPMI 1680 (manufactured by Iwaki) to which 10% of fetal bovine serum was added. SK-OV-3 was incubated using a McCoy's 5a medium (manufactured by Gibco) to which 10% of fetal bovine serum was added. J82 was incubated using an MEM medium (manufactured by Gibco) to which 10% of fetal bovine serum was added. HeLa Tet-On cells were purchased from Clontech and incubated using a DMEM medium (manufactured by Gibco) to which 10% Tet System Approved Fetal Bovine Serum (manufactured by Clontech) was added.

### (Cdk4 inhibiting action)

### (1) Purification of cyclin D2-Cdk4

Firstly, Cdk4 and cDNA of a fused product of its activating factor cyclin D2 with glutathione S transferase were incorporated into a baculovirus to prepare a recombined baculovirus. The prepared vaculovirus was infected to insect cells Sf9 to highly express as an active complex with a fused product of cyclin D2 and glutathione S transferase. The cells were recovered and solubilized, the active complex was adsorbed with glutathione Sepharose and a complex of cyclin D2-Cdk4 was recovered by a precision protease and purified by an HPLC column chromatography (EMBO J., 1996, volume 15, pages 7060 to 7069).

### (2) Measurement of activity of cyclin D2-Cdk4

In the measurement of cyclin D2-Cdk4, a synthetic peptide (Arg-Pro-Pro-Thr-Leu-Ser-Pro-Ile-Pro-His-Ile-Pro-Arg, SEQ ID No. 5) corresponding to the 775th to 787th amino acids of an RB protein was used as a substrate therefor (EMBO J., 1996, volume 15, pages 7060 to 7069).

The reaction was carried by a method of Kitagawa, et al. (Oncogene, 1992, volume 7, pages 1067 to 1074) with a partial modification. Volume of the reaction solution was 21.1 µl and composition of the reaction buffer (R buffer) was made 20 mM Tris hydrochloride buffer (pH 7.4)/10 mM magnesium chloride/4.5 mM 2-mercaptoethanol/1 mM ethyleneglycol bis(beta-aminoethyl ether)-N,N,N',N'-tetraacetic acid (EGTA). To the R buffer were added pure cyclin D2-Cdk4, 100 µmol of a substrate peptide, 50 µmol of a non-labeled adenosine triphosphate (ATP) and 1 µCi of [gamma³³P]labeled ATP (2,000 to 4,000 Ci/mmol; manufactured by Daiichi Kagaku Yakuhin) followed by subjecting to a reaction at 30°C for 45 minutes. After that, the reaction was stopped by addition of 10 µl of 350 mM phosphate buffer to the reaction system. The substrate peptide was adsorbed with a P81 paper filter 96-well plate and washed for several times with 75 mM phosphate buffer and the radiation activity was measured by a liquid scintillation counter.

Addition of a test compound to the reaction system was conducted in such a manner that, firstly, a series of diluted compound with dimethyl sulfoxide (DMSO) was prepared and 1.1 µl thereof was added. DMSO (1.1 µl) was added to the reaction system and used as a control.

### (Cdk6 inhibiting action)

### (1) Purification of cyclin D2-Cdk6

The same method as in the case of cyclin D2-Cdk4 was carried out except that Cdk6 and cDNA of a fused product of its activating factor cyclin D2 with glutathione S transferase were incorporated into a baculovirus expression vector to prepare a recombined baculovirus.

### (2) Measurement of cyclin D2-Cdk6

The same method as in the case of cyclin D2-Cdk4 was carried out except that cyclin D2-Cdk6 was used instead of cyclin D2-Cdk4.

### (Cdk2 inhibiting action)

### (1) Purification of cyclin A-Cdk2

The same method as in the case of cyclin D2-Cdk4 was carried out except that Cdk2 and cDNA of a fused product of its activating factor cyclin A with glutathione S transferase were incorporated into a baculovirus expression vector to prepare a recombined baculovirus.

### (2) Measurement of activity of cyclin A-Cdk2

Measurement of activity of cyclin A-Cdk2 was carried out by the same method as in the measurement of activity of cyclin D2-Cdk4 except that a synthetic peptide (Ala-Lys-Ala-Lys-Lys-Thr-Pro-Lys-Lys-Ala-Lys-Lys: SEQ ID No. 6) was used as a substrate and cyclin A-Cdk2 was used instead of cyclin D2-Cdk4.

### (Cdk1 inhibiting action)

### (1) Purification of cyclin B-Cdk1

The same method as in the case of cyclin D2-Cdk4 was carried out except that Cdk1 and cDNA of a fused product of its activating factor cyclin B with glutathione S transferase were incorporated into a baculovirus expression vector to prepare a recombined baculovirus.

### (2) Measurement of cyclin B-Cdk1

The same method as in the measurement of activity of cyclin A-Cdk2 was carried out except that cyclin B-Cdk1 was used instead of cyclin A-Cdk2.

### (Cdk7 inhibiting action)

### (1) Purification of cyclin H-Cdk7

The same method as in the case of cyclin D2-Cdk4 was carried out except that Cdk7 and cDNA of a fused product of its activating factor cyclin H with glutathione S transferase were incorporated into a baculovirus expression vector to prepare a recombined baculovirus.

### (2) Measurement of activity of cyclin H-Cdk7

Measurement of activity of cyclin H-Cdk7 was carried out by the same method as in the measurement of activity of cyclin D2-Cdk4 except that a synthetic peptide (Tyr-Ser-Pro-Thr-Ser-Pro-Thr-Tyr-Ser-Pro-Thr-Ser-Pro-Thr- Tyr-Ser-Pro-Thr-Ser-Pro-Thr-Tyr-Ser-Pro-Thr-Ser-Pro-Thr: SEQ ID No. 7) was used as a substrate and cyclin H-Cdk7 was used instead of cyclin D2-Cdk4.

### (Measurement of cell growth suppressing action)

Suppressive action for cell growth was measured by a sulforhodamine B dye-staining method. Each 100 µl of culture medium for each cells containing 2×10³ cells each as living cell numbers was placed in a 96-well dish for cell incubation and incubation was carried out for one night. On the next day, a dilution series with DMSO was prepared from a solution of the compound in DMSO. After that, the dilution series or DMSO only as a control where no drug was added thereto was added to a culture medium for each cell. Finally, each 100 µl of the dilution series of each drug or DMSO only was added to the cell incubated in a 96-well dish followed by incubating for three days more.

To each well was added each 20 µl of WTS-8 (manufactured by Kishida Kagaku) and incubated for 2 hours more and optical concentration thereof at 450 nm was measured using 650 nm as a reference wavelength and compared with the control group. The concentration by which cell growth was inhibited to an extent of 50% by the compound A was defined as IC50 and the values were determined.

Inhibiting activity *in vitro* of a CDK inhibitor (hereinafter, it will be referred to as the compound A) used in this Example is shown in Table 1. It has been able to be confirmed from Table 1 that the compound A inhibits the kinase activity of a CDK family in low concentrations. It has been found on the contrary that no inhibiting action is available in low concentrations to the kinase which is other than the CDK family. Accordingly, the compound A is believed to be an inhibitor selective to CDK.

**[Table 1]**

| CDKs | IC50 (nM) | Other kinases | IC50 (nM) |
|---|---|---|---|
| CDK4 | 10 | ERK1 | >1000 |
| CDK6 | 13 | ERK2 | >1000 |
| CDK2 | 16 | PKA | >1000 |
| CDC2 | 13 | PKC | >1000 |
| CDK7 | 16 | | |

Effect of the compound A to phosphorylation of an Rb protein which is a substrate in CDK cells was also investigated.

Thus, the phosphorylated state of the Rb protein during the treatment with a drug was checked by a western blotting using an antibody which specifically recognizes a phosphorylated Rb protein.

Firstly, the cells were placed in a 6-well dish for cell incubation and incubated for one night. On the next day, the compound A (final concentrations: 3, 10, 30 and 100 nM) and DMSO as a control to which no drug was added was added to the medium for incubation of each cell. After treating with a drug for 24 hours, the cells were recovered. A solution of the ground cells was separated by means of 7.5% SDS polyacrylamide gel electrophoresis and transcribed from the gel to Immobilon PVDF membrane (manufactured by Millipore). The PVDF membrane was dipped in a primary antibody solution containing anti-Ser 780 (manufactured by Cell Signaling) which is a specific antibody to phosphorylated Rb protein and impregnated for a whole day and night at 4°C. After the impregnation, the PVDF membrane was dipped in a secondary antibody solution containing HRP-conjugated goat antirabbit IgG and impregnated for one hour at 4 °C. After the impregnation, it was washed with a TBS-T buffer. After that, an X-ray film was exposed with chemiluminescence using an ECL (manufactured by Amersham) to check the existing amount of the aimed antigen.

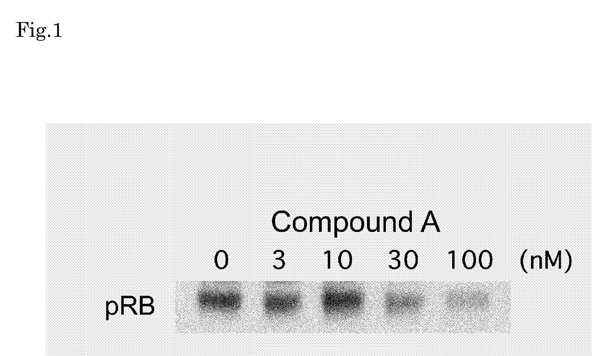

The result is as shown in Fig. 1. A band in the gel shows a phosphorylated Rb protein (pRB). As a result, as compared with the Rb protein in the cells treated with DMSO which was for a control where no drug was added, phosphorylation of the Rb protein lowered in a concentration-depending manner when treated with the compound A. Accordingly, it is now apparent that, even in cells, the compound A has an activity of inhibiting the CDK.

### (Method for detection of apoptosis)

Induction of apoptosis was analyzed using a flow cytometry and checked by means of an increase in SubG1 fraction. Each cell was placed in a 6-well dish for cell incubation and incubated for one night. On the next day, the compound A and DMSO as a control to which no drug was added was added to the medium for incubation of each cell. After treating with a drug for 3 days, the cells were recovered. DNA of the cells was stained with a Cycle TESTTM PLUS DNA Reagent Kit (manufactured by Becton Dickinson) and analyzed using a flow cytometry.

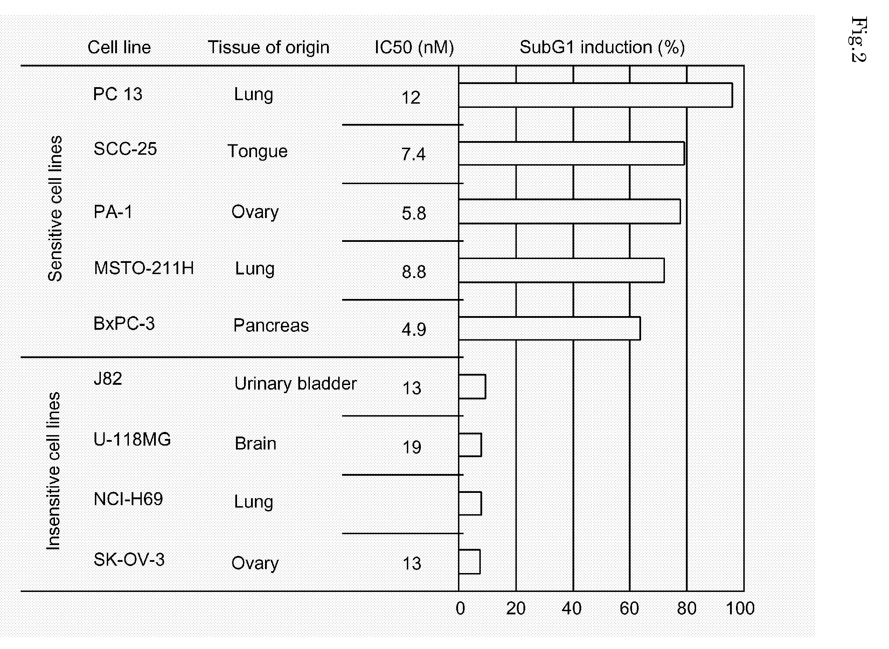

Results of checking the cell growth-inhibiting activity and the apoptosis-inducing activity of the compound A are shown in Fig. 2. With regard to the cell growth-inhibiting activity, a concentration where cell growth was inhibited by the compound A to an extent of 50% was defined IC50 and the value was determined. Induction of apoptosis was analyzed using a flow cytometry and checked by an increase in a SubG1 fraction. In the drawing, IC50 means the above-mentioned cell growth-inhibiting activity and subG1 induction means the rate of apoptosis induction.

IC50 of the compound A was nearly in the same extent for various cells. On the contrary, induction of apoptosis by the compound A showed different value for each cell. From the above, it is now apparent that the compound A has the same growth-inhibiting activity to a broad range of cells and that, after the growth inhibition, there were cells where apoptosis was and was not induced.

### (Analysis of gene expression by microarray)

Total RNA was constructed from each cell strain using an RNeasy total RNA isolation kit (manufactured by Qiagen) and labeled with biotin using T7 RNA polymerase (manufactured by Enzo Diagnostics). The labeled RNA was hybridized to Gene Chip TM (manufactured by Affymetrix) HG-U95 chip containing about 12,000 human gene probe set. Methods for hybridization and chip washing were conducted in accordance with the protocol of Affymetrix Fluidix Station.

In order to find the gene useful for assumption of resistance to a CDK inhibitor, investigations were conducted for genes having different expression amounts between a cell strain which is apt to cause apoptosis by a CDK inhibitor and a cell strain which is not apt to do that. Twenty kinds of "round-robin" comparisons were carried out among five types of drug-sensitive strains (BxPC III, MSTO-211H, PA-1, SCC-25 and PC13) and four types of drug-resisting strains (SK-OV-III, NCI-H69, U118-MG and J82) and genes satisfying the following criteria were picked up as genes having a difference in expression amount between sensitive and resisting strains. For the comparison of each combination, Microarray Analysis Suite (Microarray Analysis Suite; manufactured by Affymetrix) was used. As a criterion for the selection of gene, the following criterion 1 was provided.

Criterion 1: Gene in which expression in sensitive strain is higher than that in resisting strains in 16 or more comparisons in the 20 or gene in which expression in resisting strains is higher than that in sensitive strains in 16 comparisons or more.

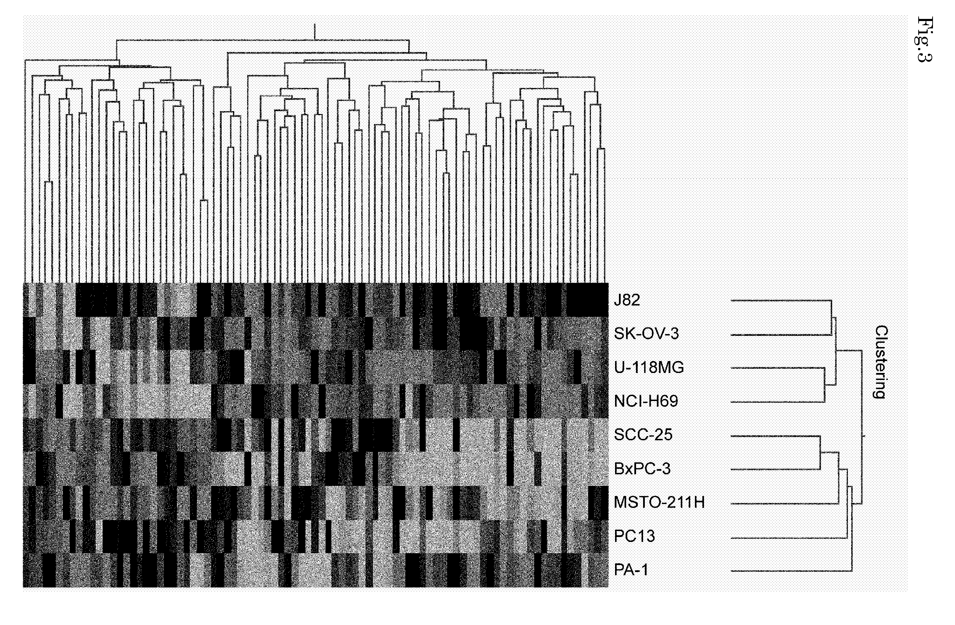

There were 87 genes which satisfied the criterion and a clustering was carried out using those genes (Fig. 3). The clustering was conducted using Gene Spring (manufactured by Silicon Genetics). Cell strain and gene are clustered in length and breadth directions, respectively. The clustering is displayed according to a method of pattern classification in such a manner that cell strains and genes where patterns are similar are located near on tree-shaped charts. In this clustering, it is understood that cell strains are classified into a group where sensitivity is strong and a group where it is weak. From the above, it is believed that assumption of resistance to a CDK inhibitor is possible from the expression amount of those genes.

However, according to such a condition only, there is a possibility that not only a gene having difference in expression amount between sensitive strain and resisting strain but also a gene which is expressed in a tissue-specific manner may be selected. For example, when there are many cells derived from stomach in a resisting strain and when there are many cells derived from lung in a sensitive strain, gene which is related to drug resistance is not selected but gene which is tissue-specifically expressed is selected. Accordingly, genes satisfying the following criterion 2 were selected from the 87 genes.

Criterion 2: Genes in which expression in sensitive strains is higher than that in resisting strains and gene in which expression in resisting strains is higher than that in sensitive strains in all of three comparisons, i.e. comparisons of lung-derived sensitive strains with resisting strains (PC13 vs. NCI-H69 and MSTO-211H vs. NCI-H69) and comparison of ovarium-derived cell strains (PA-1 vs. SK-OV-III).

As a result, there were found 15 genes where higher expression was noted in the sensitive strains than in the resisting strains and 27 genes where higher expression was noted in the resisting strains that in the sensitive strains (Table 2). Genes which participate in cell cycle/elongation, signal transduction, transcription control, etc. are included in those and the difference in the expression as such is believed to be a cause for the difference in drug resistance. Particularly, as a gene having a deep relation to CDK, it has been clarified that expression of p18 (one of CDK inhibitors) gene is higher in resisting strains than in resisting strains.

**[Table 2]**

| (a) positively correlated with sensitivity | | |
|---|---|---|
| Probe Set | Sequence | Name |
| 33396_at | U12472 | glutathione S-transferase pi |
| 33451_s_at | AI526079 | EST |
| 40081_at | L26232 | phospholipid transfer protein |
| 829_s_at | U21689 | glutathione S-transferase pi |
| 32314_g_at | M75165 | tropomyosin 2 (beta) |
| 32747_at | X05409 | aldehyde dehydrogenase 2 family (mitochondrial) |
| 33294_at | D29958 | KIAA0116 protein |
| 37619_at | D42084 | methionyl aminopeptidase 1 |
| 40764_at | M22632 | glutamic-oxaloacetic transaminase 2, mitochondrial |
| 34570_at | S79522 | ribosomal protein S27a |
| 34592_at | M13932 | ribosomal protein S17 |
| 34593_g_at | M13932 | ribosomal protein S17 |
| 35785_at | W28281 | GABA(A) receptor-associated protein like 1 |
| 36636_at | M12267 | ornithine aminotransferase (gyrate atrophy) |
| 37883_i_at | AI375033 | hypothetical protein AF038169 |
| 39839_at | M24069 | cold shock domain protein A |
| 40756_at | AF081280 | nucleophosmin/nucleoplasmin, 3 |

| (b) negatively correlated with sensitivity | | |
|---|---|---|
| Probe Set | Sequence | Name |
| 32215_i_at | AB020685 | Rho-related BTB domain containing 3 |
| 32168_s_at | U85267 | Down syndrome critical region gene 1 |
| 1488_at | L77886 | protein tyrosine phosphatase, receptor type, K |
| 32043_at | AF098462 | stanniocalcin 2 |
| 32171_at | AL080102 | eukaryotic translation initiation factor 5 |
| 32214_at | AF003938 | thioredoxin-like, 32kDa |
| 32542_at | AF063002 | four and a half LIM domains 1 |
| 32628_at | D28118 | zinc finger protein 161 |
| 33362_at | AF094521 | CDC42 effector protein (Rho GTPase binding) 3 |
| 33877_s_at | AB028990 | exocyst complex component 7 |
| 33905_at | AF072242 | methyl-CpG binding domain protein 2 |
| 34719_at | AB020645 | glutaminase |
| 350_at | D28118 | zinc finger protein 161 |
| 36053_at | AF041248 | cyclin-dependent kinase inhibitor 2C (p18) |
| 36532_at | AF039945 | synaptojanin 2 |
| 38990_at | AL031178 | F-box only protein 9 |
| 39093_s_at | Y12059 | bromodomain containing 4 |
| 39250_at | X78351 | nephroblastoma overexpressed gene |
| 39470_at | AL049974 | cDNA DKFZp564B222 |
| 39756_g_at | Z93930 | X-box binding protein 1 |
| 39806_at | AL050261 | ASF1 anti-silencing function 1 homolog A |
| 40167_s_at | AF038187 | WD repeat and SOCS box containing protein 2 |
| 40478_at | AL021396 | hypothetical protein DJ971N18.2 |
| 40710_at | D86322 | calmegin |
| 40847_at | AB018293 | flavoprotein oxidoreductase MICAL2 |
| 40848_g_at | AB018293 | flavoprotein oxidoreductase MICAL2 |
| 41476_at | N36926 | guanine nucleotide binding protein, alpha 11 (Gq class) |
| 41489_at | M99435 | transducin-like enhancer of split 1 |
| 564_at | M69013 | guanine nucleotide binding protein, alpha 11 (Gq class) |
| 901_g_at | L41349 | phospholipase C, beta 4 |

### (Western blotting)

An extract of each incubated cells was separated by a 12.5% SDS polyacrylamide gel electrophoresis and transcribed from the gel to an Immobilon PVDF membrane (manufactured by Millipore). The PVDF membrane was dipped in a primary antibody solution containing an anti-p18 (manufactured by Santa Cruz Biotechnology) and permeated at 4°C for a whole day and night. After the impregnation, the PVDF membrane was dipped in a secondary antibody solution containing HRP-conjugated goat antirabbit IgG and impregnated for one hour at 4 °C. After the impregnation, it was washed with a TBS-T buffer and, after that, chemiluminescence was exposed to an X-ray film using ECL (manufactured by Amersham) to detect the existing position of the aimed antigen.

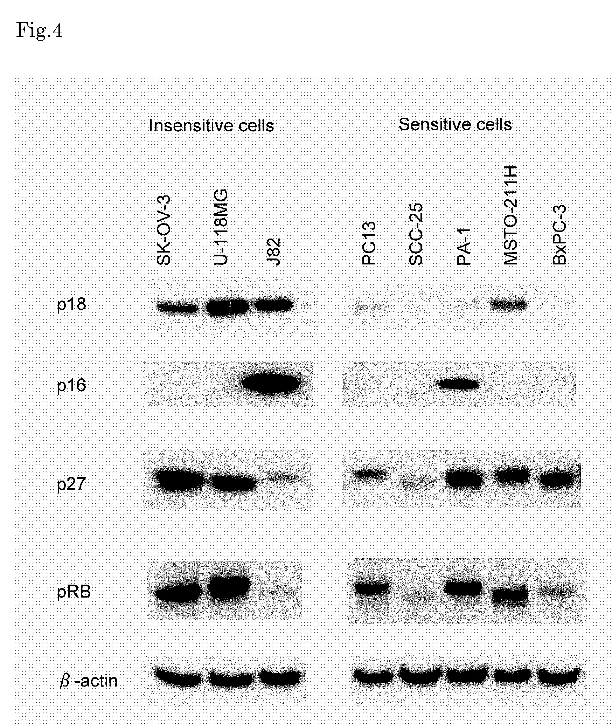

Fig. 4 shows the result where expression amounts of a CDK inhibitors and a phosphorylated Rb proteins were checked in the cells in which apoptosis was induced by a compound A and is not. pRB in the drawing shows a phosphorylated Rb protein and β-actin is an internal standard of protein amount extracted from each cell used for the analysis.

Here, CDK inhibitors are cancer-suppressing genes. As a result of a decrease in their expression, it has been known that cells are overgrown resulting in carcinogenesis. It has been believed that cells becoming cancerous due to a decrease in the expression of a CDK inhibition protein have high sensitivity to CDK inhibitors.

From the above result, it is noted that expression amount of p 18 is low in all cells where apoptosis is induced by a CDK inhibitor. From the above, it has become apparent that the cells having a low expression amount of p18 have a high sensitivity to a CDK inhibitor and that the cells having a high expression amount of p18 have a low sensitivity to a CDK inhibitor.

### (Preparation of strain holding the plasmid stably)

For an object of investigating the correlation between sensitivity to the compound A to expression amount of p18, cells (p18 IND HeLa) where expression of p18 is able to be inducted by doxycyclin were established. Thus, pTRE2hyg FLAG-p18 vector was introduced into HeLa cells using Flunege 6. After the introduced cells were incubated for 24 hours, incubation was conducted so as to make cell numbers about one-fiftieth and incubation was conducted in the presence of 0.3 mg/ml of hygromycin B (manufactured by Invitrogen) for about two weeks. Cells wherein colonies were formed were isolated by a cylinder cloning method.

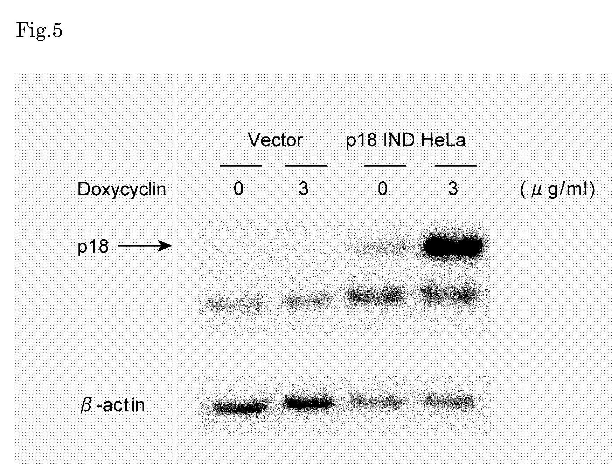

Result where the correlation between sensitivity to the compound A and expression amount of p18 was checked using the recombinant cells established as such is shown in Fig. 5. In the drawing, "vector" means a control cell into which pTRE2hyg FLAG-vector (into which no p18 is inserted) was introduced and "p 18 IND HeLa" means a cell where pTRE2hyg FLAG-p18 vector is introduced into HeLa cell and expression of p18 by doxycyclin is able to be induced.

In the control cell (vector), there was no change in expression amount of p18 by doxycyclin. On the contrary, expression of p18 is induced upon addition of 3 µg/µl of doxycyclin in the case of p18 IND HeLa cell. Accordingly, it has been confirmed that cells (p18 IND HeLa) where expression of p18 is able to be induced is able to be established.

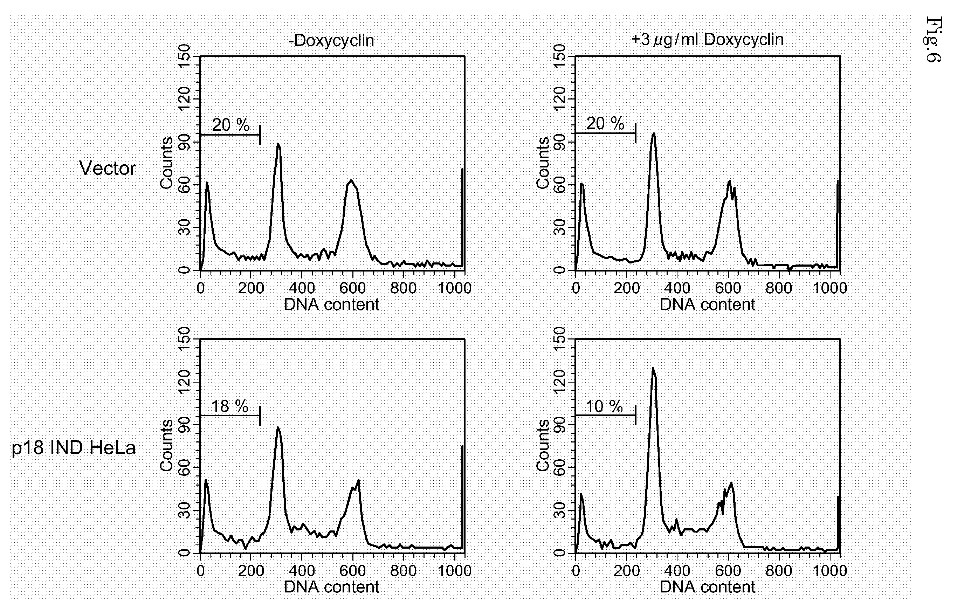

Then the correlation between sensitivity to the compound A and expression amount of p 18 was investigated. The result is as shown in Fig. 6. The left two graphs are the results for the cells to which no doxycyclin was added (-Doxycyclin) while the right two graphs are the results for the cells to which doxycyclin was added in a concentration of 3 µg/ml (+3 µg/ml Doxycyclin). In the control cells (Vector), expression amount of p18 does not change by doxycyclin and apoptosis induced by the compound A does not change as well. On the contrary, it has been noted that, in p18 IND HeLa cells, apoptosis is suppressed to an extent of 10% from 18% when expression of p18 is induced by addition of 3 µg/µl of doxycyclin. Accordingly, it has become apparent that there is a correlation between sensitivity to the compound A and expression amount of p18.

From the above, it has become apparent that there is a correlation between the sensitivity to CDK inhibitors and expression amount of p18. Accordingly, it is now apparent that checking the expression amount of p 18 is very useful for searching the diseases (particularly those of a cancer type) where CDK inhibitors achieve a therapeutic effect.

### Example 2: Experiment for expression of CDK inhibiting protein

### (Microarray analysis)

In all experiments, Affymetrix HG-U95 Av2 Gene Chip was used.

Firstly, virus vector in which p16 or p27 gene was incorporated was transfected to HCT 116 cell and compared with the cell (control) to which a virus vector where lacZ was incorporated was transfected and gene in which expression was changed by an excessive expression of p16 and p27 was checked at four time points of 24, 36, 48 and 72 hours. In order to measure the fold change, a comparative analysis of Affymetrix Microarray Suite, version 5.0 (GeneChip Software MAS-5.0) was used. In that case, when the GeneChip Software was used for the measurement of expression amount of gene my microarray, each of the information "Signal" and "Detection call" was calculated to each probe set. When the expression amount detected in a probe set of perfect match is more than the expression amount detected by a mismatch probe set which is in a pair thereof in a p-value of less than 0.04, the value of "present" (P) is given to "Detection call". On the contrary, when p-value is between 0.04 and 0.06, the value of "absent" (A) is given in case "marginal" (M) is not less than 0.06. When a Comparative Analysis of GeneChip Software is used, difference in expression amounts of gene between the two samples is measured and the values of "Signal Log Ratio" (log 2 fold change) and "Change call" are calculated. With regard to the values for "Change call", five values of "Increase" (I), "Decrease" (D), "Marginal Increase/Decrease" (MI/MD) and "No Change" (NC) are given.

In the experiment at this time, the following criteria were firstly used for extracting the gene which changed the expression by influence of CDK4 inhibition.

(a): In any one or more point(s) in the four time points, "Signal Log Ratio" when cells where p16 and p27 are excessively expressed are compared with the control is not less than 0.6 and "Change call" is "I" or "MI" or the "Signal Log Ratio" is not more than 0.6 and "Change call" is "D" or "MD".

(b): All of "Detection call" in the cell where p27 is excessively expressed and where lacZ is transfected are not "A".

(c): The case where "Detection call" in the cell where p16 is excessively expressed and where lacZ is transfected are "A" in all time points is excluded.

After that, the following criteria were used for extracting the gene which changes the expression by influence of CDK 2 inhibition.

(a): "Signal Log Ratio" when the cell where p27 is excessively expressed and the control are compared is not less than 0.6 in any time point and "Change call" is "I" or "MI" or the above is not more than 0.6 and "Change call" is "D" or "MD".

(b): "Signal Log Ratio" when the cell where p16 is excessively expressed and the control are compared is -0.3 to 0.3 in all time points.

(c): The case where "Detection call" in the cell where p27 is excessively expressed an the cell where lacZ is transfected is "A" in all time points is excluded.

### (Principal component analysis: PCA)

Gene which was extracted as a gene which changes the expression by the influence of CDK4 inhibition was subjected to a principal component analysis using SAS release 8.2 which is a software package for statistic analysis. In order to indicate the analytic result on a graphical display, GeneSpirng 6 was used. For the calculation, log 2 ratio was used as it was and no normalization was conducted.

It was checked how the gene which was selected as a result of application of the principal component analysis for the gene where 1.5-fold or more changes in expression was observed by inhibition of CDK4 will be grouped depending upon expression pattern.

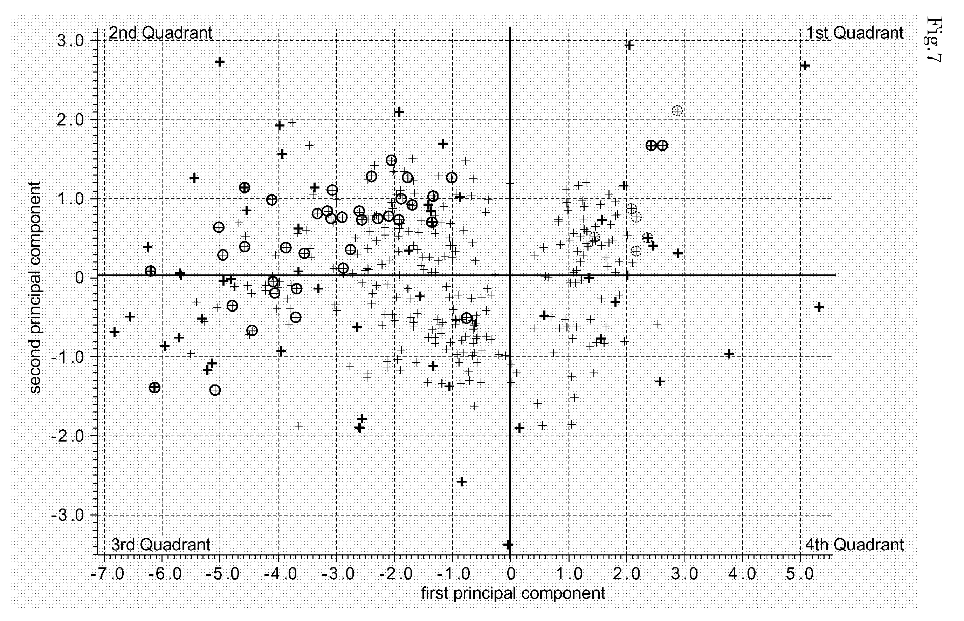

The result is shown in Fig. 7. In the graphs of Fig. 7, genes where expression increased during 48 to 72 hours, genes where expression decreased during 24 to 36 hours, genes where expression decreased during 48 to 72 hours and genes where expression increased during 24 to 36 hours were concentrated to the first quadrant (both x and y are positive numbers), to the second quadrant (x is negative and y is positive), to the third quadrant (both x and y are negative numbers) and to the fourth quadrant, respectively. Thus, it is shown that genes which cause changes in expression by CDK4 inhibition were able to be classified into four groups depending upon the changing pattern in expression.

Lists (for the genes of the first quadrant only; 1.5-fold or more) of genes where expression changes to an extent of 2.0-fold or more by inhibition of CDK4 (excessive expression of p16 and p27) are shown in Tables 3 to 6.

**[Table 3]**

| probe | ACC | SYMBOL | GENE NAME |
|---|---|---|---|
| 1st Quadrant* (60 probes, 58 genes) | | | |
| mitotic cell cycle | | | |
| 2021_s_at | M73812 | CCNE1 | cyclin E1 |
| 41060_at | M74093 | CCNE1 | cyclin E1 |
| 1274_s_at | L22005 | CDC34 | cell division cycle 34 |
| 38115_at | AF055479 | PDAP2 | PDGFA associated protein 2 |

| DNA metabolism | | | |
|---|---|---|---|
| 508_at | U43923 | SUPT4H1 | suppressor of Ty 4 homolog 1 (S. cerevisiae) |
| 35576_f_at | AL009179 | H2BFC | H2B histone family, member C |
| 36347_f_at | AA873858 | H2BFD | histone 1, H2bn |
| 31523_f_at | Z80780 | H2BFH | histone 1, H2be |
| 33835_at | AB018264 | TSPYL4 | TSPY-like 4 |

| induction of apoptosis | | | |
|---|---|---|---|
| 1114_at | M22490 | BMP4 | bone morphogenetic protein 4 |
| 38115_at | AF055479 | PDAP2 | PDGFA associated protein 2 |
| 1736_at | M62402 | IGFBP6 | insulin-like growth factor binding protein 6 |
| 1712_s_at | U17743 | MAP2K4 | mitogen-activated protein kinase kinase 4 |
| 34767_at | AI670788 | MOAP1 | modulator of apoptosis 1 |
| 32737_at | M64595 | RAC2 | ras-related C3 botulinum toxin substrate 2 (rho family, smallGTP binding protein Rac2) |

| protein biosynthesis | | | |
|---|---|---|---|
| 1114_at | M22490 | BMP4 | bone morphogenetic protein 4 |
| 40515_at | AF035280 | EIF2B2 | eukaryotic translation initiation factor 2B, subunit 2 beta |
| 1644_at | U36764 | EIF3S2 | eukaryotic translation initiation factor 3, subunit 2 beta |
| 32230_at | U39067 | EIF3S2 | eukaryotic translation initiation factor 3, subunit 2 beta |
| 1736_at | M62402 | IGFBP6 | insulin-like growth factor binding protein 6 |
| 39004_at | AI432190 | MRPS11 | mitochondrial ribosomal protein S11 |
| 549_at | S80343 | RARS | arginyl-tRNA synthetase |

| RNA processing | | | |
|---|---|---|---|
| 34286_at | AB020623 | BCAS2 | breast carcinoma amplified sequence 2 |
| 32790_at | D59253 | NCBP2 | nuclear cap binding protein subunit 2, 20kDa |
| 38380_at | Y18863 | POP4 | POP4 (processing of precursor, S. cerevisiae) homolog |
| 37936_at | Al184802 | PRPF4 | PRP4 pre-mRNA processing factor 4 homolog (yeast) |

| Miscellaneous | | | |
|---|---|---|---|
| --- | | --- | -- (37 probes, 37 genes) -- |

**[Table 4]**

| probe | ACC | SYMBOL | GENE NAME |
|---|---|---|---|
| 2nd Quadrant (20 probes, 20 genes) | | | |
| DNA replication and chromosome cycle | | | |
| 604_at | L78833 | BRCA1 | breast cancer 1, early onset |
| 40041_at | AF017790 | HEC | highly expressed in cancer, rich in leucine heptad repeats |
| 32682_at | U09087 | TMPO | thymopoietin |
| 2003_s_at | U28946 | MSH6 | mutS homolog 6 (E. coli) |
| 37646_at | D26018 | POLD3 | polymerase (DNA directed), delta 3 |
| 38834_at | D87448 | TOPBP1 | topoisomerase (DNA) II binding protein |

| mitotic cell cycle | | | |
|---|---|---|---|
| 2003_s_at | U28946 | MSH6 | mutS homolog 6 (E. coli) |
| 39420_at | S62138 | FUS | fusion, derived from t(12;16) malignant liposarcoma |
| 1803_at | X05360 | CDC2 | cell division cycle 2, G1 to S and G2 to M |
| 35249_at | AF091433 | CCNE2 | cyclin E2 |
| 40041_at | AF017790 | HEC | highly expressed in cancer, rich in leucine heptad repeats |
| 418_at | X65550 | MKI67 | antigen identified by monoclonal antibody Ki-67 |
| 1651_at | U73379 | UBE2C | ubiquitin-conjugating enzyme E2C |
| 40726_at | U37426 | KIF11 | kinesin family member 11 |
| 356_at | AB017430 | KIF22 | kinesin family member 22 |
| 37171_at | X67155 | KIF23 | kinesin family member 23 |

| regulation of cell cycle | | | |
|---|---|---|---|
| 604_at | L78833 | BRCA1 | breast cancer 1, early onset |
| 39420_at | S62138 | FUS | fusion, derived from t(12;16) malignant liposarcoma |
| 418_at | X65550 | MKI67 | antigen identified by monoclonal antibody Ki-67 |
| 2003_s_at | U28946 | MSH6 | mutS homolog 6 (E. coli) |
| 1651_at | U73379 | UBE2C | ubiquitin-conjugating enzyme E2C |
| 35249_at | AF091433 | CCNE2 | cyclin E2 |
| 1803_at | X05360 | CDC2 | cell division cycle 2, G1 to S and G2 to M |
| 38834_at | D87448 | TOPBP1 | topoisomerase (DNA) II binding protein |

| response to DNA damage stimulus | | | |
|---|---|---|---|
| 39420_at | S62138 | FUS | fusion, derived from t(12;16) malignant liposarcoma |
| 1803_at | X05360 | CDC2 | cell division cycle 2, G1 to S and G2 to M |
| 38834_at | D87448 | TOPBP1 | topoisomerase (DNA) II binding protein |
| 2003_s_at | U28946 | MSH6 | mutS homolog 6 (E. coli) |
| 604_at | L78833 | BRCA1 | breast cancer 1, early onset |
| 33145_at | X99226 | FANCA | Fanconi anemia, complementation group A |
| 41479_s_atAF029670 RAD51C RAD51 homolog C (S. cerevisiae) | | | |

| DNA metabolism | | | |
|---|---|---|---|
| 604_at | L78833 | BRCA1 | breast cancer 1, early onset |
| 33145_at | X99226 | FANCA | Fanconi anemia, complementation group A |
| 39969_at | AA255502 | HIST1 H4C | histone 1, H4c |
| 2003_s_at | U28946 | MSH6 | mutS homolog 6 (E. coli) |
| 37646_at | D26018 | POLD3 | polymerase (DNA directed), delta 3 |
| 41479_s_atAF029670 RAD51C RAD51 homolog C (S. cerevisiae) | | | |
| 38834_at | D87448 | TOPBP1 | topoisomerase (DNA) II binding protein |

| Miscellaneous | | | |
|---|---|---|---|
| 40412_at | AA203476 | PTTG1 | pituitary tumor-transforming 1 |
| 39677_at | D80008 | KIAA0186 | KIAA0186 gene product |
| 37351_at | X90858 | UPP1 | uridine phosphorylase 1 |

**[Table 5]**

| probe | ACC | SYMBOL | GENE NAME |
|---|---|---|---|
| 3rd Quadrant (21 probes, 20 genes) | | | |
| DNA replication and chromosome cycle | | | |
| 41081_at | AF053305 | BUB1 | BUB1 budding uninhibited by benzimidazoles 1 homolog (yeast) |
| 1945_at | M25753 | CCNB1 | cyclin B1 |
| 34736_at | M25753 | CCNB1 | cyclin B1 |
| 37302_at | U30872 | CENPF | centromere protein F, 350/400ka (mitosin) |
| 1376_at | M36067 | LIG1 | ligase I, DNA, ATP-dependent |
| 798_at | X74330 | PRIM1 | primase, polypeptide 1, 49kDa |

| mitotic cell cycle | | | |
|---|---|---|---|
| 1376_at | M36067 | LIG1 | ligase I, DNA, ATP-dependent |
| 798_at | X74330 | PRIM1 | primase, polypeptide 1, 49kDa |
| 36837_at | U63743 | KIF2C | kinesin family member 2C |
| 41081_at | AF053305 | BUB1 | BUB1 budding uninhibited by benzimidazoles 1 homolog (yeast) |
| 37302_at | U30872 | CENPF | centromere protein F, 350/400ka (mitosin) |
| 1945_at | M25753 | CCNB1 | cyclin B1 |
| 34736_at | M25753 | CCNB1 | cyclin B1 |
| 32263_at | AL080146 | CCNB2 | cyclin B2 |
| 1599_at | L25876 | CDKN3 | cyclin-dependent kinase inhibitor 3 (CDK2-associated dual specificity phosphatase) |
| 39374_at | AL022325 | GTSE1 | G-2 and S-phase expressed 1 |

| regulation of cell cycle | | | |
|---|---|---|---|
| 41081_at | AF053305 | BUB1 | BUB1 budding uninhibited by benzimidazoles 1 homolog (yeast) |
| 1945_at | M25753 | CCNB1 | cyclin B1 |
| 34736_at | M25753 | CCNB1 | cyclin B1 |
| 32263_at | AL080146 | CCNB2 | cyclin B2 |
| 1599_at | L25876 | CDKN3 | cyclin-dependent kinase inhibitor 3 (CDK2-associated dual specificity phosphatase) |
| 37302_at | U30872 | CENPF | centromere protein F, 350/400ka (mitosin) |
| 35799_at | AL080081 | DNAJB9 | DnaJ (Hsp40) homolog, subfamily B, member 9 |
| 39374_at | AL022325 | GTSE1 | |

| DNA metabolism | | | |
|---|---|---|---|
| 38065_at | X62534 | HMGB2 | high-mobility group box 2 |
| 798_at | X74330 | PLIM1 | primase, polypeptide 1, 49kDa |
| 1945_at | M25753 | CCNB1 | cyclin B1 |
| 34736_at | M25753 | CCNB1 | cyclin B1 |
| 37302_at | U30872 | CENPF | centromere protein F, 350/400ka (mitosin) |
| 1376_at | M36067 | LIG1 | ligase I, DNA, ATP-dependent |

| Miscellaneous | | | |
|---|---|---|---|
| 38940_at | N58115 | AD024 | AD024 protein |
| 33309_at | AA521060 | ABHD5 | abhydrolase domain containing 5 |
| 33880_at | D89053 | FACL3 | fatty-acid-Coenzyme A ligase, long-chain 3 |
| 33162_at | X02160 | INSR | insulin receptor |
| 41481_at | X17033 | ITGA2 | integrin, alpha 2 (CD49B, alpha 2 subunit of VLA-2 receptor) |
| 39696_at | AB028974 | PEG10 | paternally expressed 10 |
| 35614_at | AB012124 | TCFL5 | transcription factor-like 5 (basic helix-loop-helix) |
| 35754_at | L40391 | TMP21 | transmembrane trafficking protein |
| 36760_at | U79277 | | Homo sapiens similar to 40S ribosomal protein S26 (LOC286153), mRNA |

**[Table 6]**

| probe | ACC | SYMBOL | GENENAME |
|---|---|---|---|
| 4th Quadrant (8 probes, 8 genes) | | | |
| DNA metabolism | | | |
| 1916_s_at | V01512 | FOS | v-fos FBJ murine osteosarcoma viral oncogene homolog |
| 38576_at | AJ223353 | H2BFB | H2B histone family, member B |
| 32819_at | AJ223352 | H2BFT | H2B histone family, member T |

| cell differentiation | | | |
|---|---|---|---|
| 1916_s_at | V01512 | FOS | v-fos FBJ murine osteosarcoma viral oncogene homolog |
| 38489_at | M60047 | HBP17 | heparin-binding growth factor binding protein |
| 547_s_at | S77154 | NR4A2 | nuclear receptor subfamily 4, group A, member 2 |

| iinflammatory response | | | |
|---|---|---|---|
| 1916_s_at | V01512 | FOS | v-fos FBJ murine osteosarcoma viral oncogene homolog |
| 1165_at | D49950 | IL18 | interleukin 18 (interferon-gamma-inducing factor) |
| 547_s_at | S77154 | NR4A2 | nuclear receptor subfamily 4, group A, member 2 |

| Miscellaneous | | | |
|---|---|---|---|
| 38373_g_at | U66042 | LOC91966 | hypothetical protein LOC91966 |
| 39075_at | AF040958 | NEU1 | sialidase 1 (lysosomal sialidase) |

In the first quadrant (a gene group where expression increased during 48 to 72 hours), five genes related to apoptosis induction were observed. Although the relation between stop of cell cycle by CDK inhibition and induction of apoptosis has not been clarified yet, there is a possibility that those genes are related thereto. In the second and the third quadrants, many genes related to cell cycle were concentrated as expected. Thus, it has become apparent that the cell cycle stops by CDK4 inhibition and that the gene related to apoptosis induced thereby is extracted.

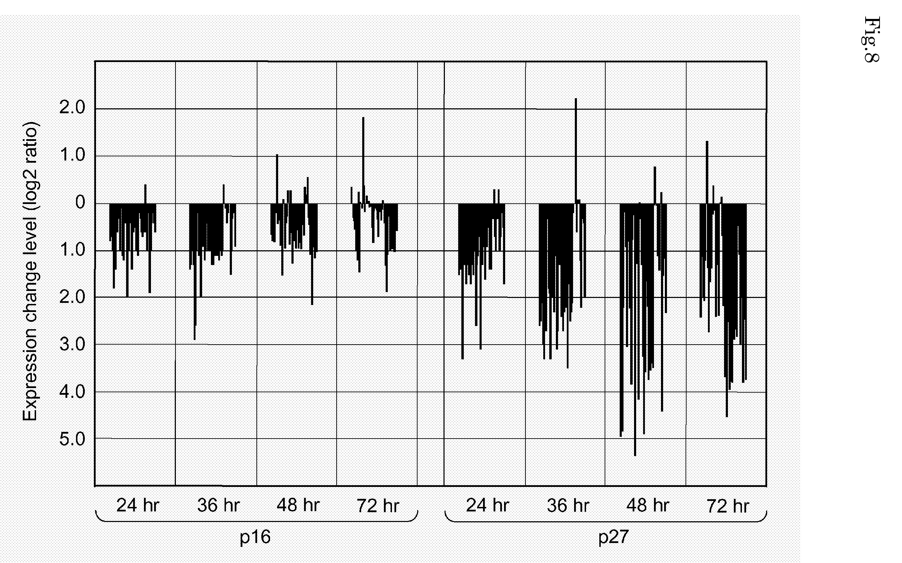

Fig. 8 shows log 2 ratio (changes in expression upon comparison of the cells to which lac Z-containing virus vector was transfected with the cells to which p16 or p27-containing virus vector was transfected) of 41 genes in total contained in the second and the third quadrants among the genes listed in Tables 3 to 6. It is noted from Fig. 8 that the decrease in expression of the selected genes is larger in p27 than in p16. Thus, it is now apparent that the influence on gene expression is different between p16 and p27.

### (Hierarchical clustering)

Calculation of a hierarchical clustering and indication on a graphical display were conducted using GeneSpring 6. Values of log 2 ratio calculated upon comparison of the cells where p16 or p27 was excessively expressed and the cells where lac Z was excessively expressed were normalized so that the average became 0 and the dispersion became 1 for each gene and the values were used for the calculation of the hierarchical clustering. Pearson's correlation coefficient was applied to a distance function.

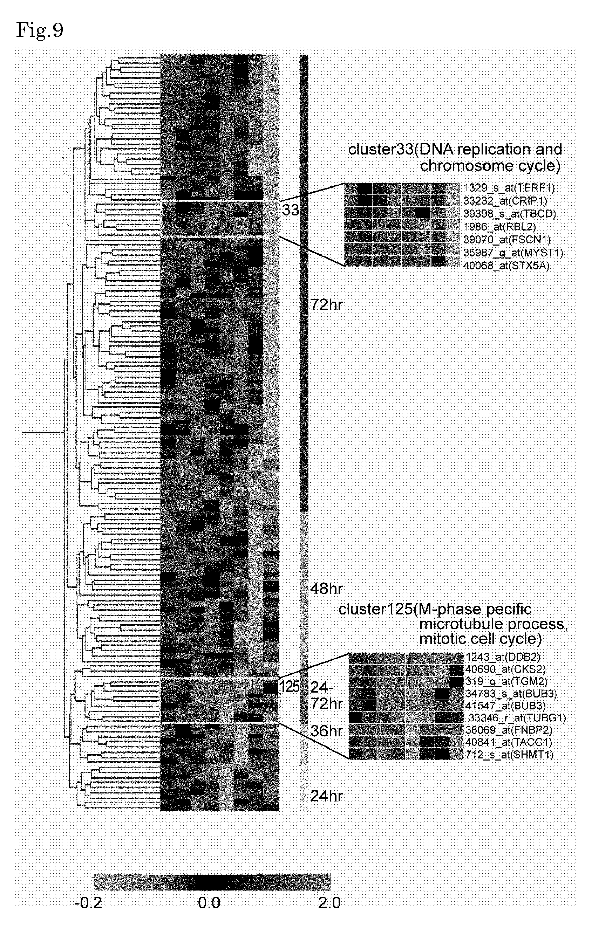

Fig. 8 shows the result where a hierarchical clustering was applied to the genes where a decrease in expression of not less than 1.5-fold was noted by inhibition of CDK2. From Fig. 8, changes in expression in accordance with time series were able to be confirmed only for the cells where p27 was excessively expressed. In the cells where p16 was excessively expressed, no change in expression took place at all in a part of them while, in the cells where p27 was excessively expressed, clusters where expression decreased in all time points of 24, 36, 48 and 72 hours were confirmed (cluster no. 125 in Fig. 9). When the genes contained in this cluster were checked in detail, genes which were related to an M-phase specific microtubule process were abundantly contained. Details of those genes are shown in Table 7.

**[Table 7]**

| cluster 125 probe | ACC | SYMBOL | GENE NAME |
|---|---|---|---|
| 1243_at | U18300 | DDB2 | damage-specific DNA binding protein 2, 48kDa |
| 40690_at | X54942 | CKS2 | CDC28 protein kinase regulatory subunit 2 |
| 319_g_at | D64142 | H1FX | H1 histone family, member X |
| 34783_s_at | AF047473 | BUB3 | BUB3 buddinguninhibited by benzimidazoles 3 homolog(yeast) |
| 41547_at | AF047472 | BUB3 | BUB3 budding uninhibited by benzimidazoles 3 homolog(yeast) |
| 33346_r_at | M61764 | TUBG1 | tubulin, gamma 1 |
| 36069_at | AB007925 | FNBP2 | formin binding protein 2 |
| 40841_at | AF049910 | TACC1 | transforming, acidic coiled-coil containing protein 1 |
| 712_s_at | HG2379-HT3997 | SHMT1 | serine hydroxymethyltransferase 1 (soluble) |

### Industrial Applicability

In accordance with the method for assumption of gene and drug sensitivity of the present invention, the knowledge for sensitivity to a CDK4 inhibitor or, particularly, that for drug resistance is now able to be assumed based on the expression amount of p16, p18 or p27 gene. It is now possible to assume the sensitivity to a CDK4 inhibitor to be administered and the pharmaceutical effect thereof only by measurement of expression of p16, p18 or p27 gene in a test tissue or a test cell such as blood separated from a test sample and it is possible to utilize to the so-called made-to-order therapy.

## Claims

1. A gene for predicting drug sensitivity, which is to be used for predicting the drug sensitivity to a CDK4 inhibitor based on the expression amount in a test tissue or a test cell, selected from the group consisting of p 16 gene, p 18 gene and p27 gene.

2. The gene for predicting drug sensitivity according to claim 1, wherein the gene selected from the group consisting of p16 gene, p18 gene and p27 gene is p18 gene.

3. The gene for predicting drug sensitivity according to claim 1 or 2, wherein the drug sensitivity is drug resistance.

4. A reagent for detection of drug sensitivity to a CDK4 inhibitor containing an antibody which specifically recognizes an antisense nucleic acid of gene selected from the group consisting of p 16 gene, p18 gene and p27 gene or protein expressed by said gene.

5. A probe for detection of drug sensitivity to a CDK4 inhibitor comprising an antisense nucleic acid of gene selected from the group consisting of p16 gene, p18 gene and p27 gene.

6. An antibody for detection of drug sensitivity to a CDK4 inhibitor comprising an antibody which specifically recognizes the protein in which gene selected from the group consisting of p 16 gene, p18 gene and p27 gene is expressed.

7. A method of predicting the drug sensitivity to a CDK4 inhibitor including
a step where expression amount of at least one gene selected from the group consisting of p16 gene, p18 gene and p27 gene in a test tissue or in a test cell is measured and
a step where the expression amount of said gene is compared with the expression amount of said gene in a healthy tissue or in a healthy cell.

8. The method of predicting according to claim 7, wherein a step where it is judged that drug sensitivity of a test tissue or a test cell to a CDK4 inhibitor is high is further included therein in case expression of the above gene in a test tissue or in a test cell is lower than expression of the above gene in a healthy tissue or in a healthy cell as a result of comparison of the expression amounts.

9. A method of predicting drug sensitivity to a CDK4 inhibitor including a step where expression of at least one gene selected from the group consisting of p16 gene, p18 gene and p27 gene in a test tissue or a test cell is detected.

10. A method of predicting according to claim 9, wherein a step where drug sensitivity of the above-mentioned test tissue or test cell to a CDK4 inhibitor is judged to be low is further included in case the above-mentioned gene is expressed as a result of the detection.

11. The method of predicting according to any of claims 7 to 10, wherein the gene is p18 gene.

12. Use of a gene selected from the group consisting of p16 gene, p18 gene and p27 gene for predicting drug sensitivity to a CDK4 inhibitor based on the expression amount in a test tissue or a test cell.
